# EUROPÄISCHE PATENTSCHRIFT

(11) **EP 0 642 506 B1**
(45) Veröffentlichungstag und Bekanntmachung des Hinweises auf die Patenterteilung: **04.09.2002**
(21) Anmeldenummer: 93910007.9
(22) Anmeldetag: 17.05.1993
(51) Int. Cl.: C07D 403/12, C07D 405/12, C07D 409/12, C07D 411/12, C07D 413/12, C07D 417/12, A01N 47/28, A01N 47/36

(54) **ZWEIFACH HETEROCYCLISCH SUBSTITUIERTE SULFONYLAMINO(THIO)CARBONYLVERBINDUNGEN**
HETEROCYCLICALLY BISUBSTITUTED SULPHONYL AMINO(THIO)CARBONYL COMPOUNDS
COMPOSES SULFONYLAMINO(THIO)CARBONYLES BISUBSTITUES EN HETEROCYCLE

(30) Priorität: 29.05.1992 DE 4217719
(43) Veröffentlichungstag der Anmeldung: 15.03.1995
(73) Patentinhaber: BAYER AG, 51368 Leverkusen (DE)
(72) Erfinder: RIEBEL, Hans-Jochem, D-5600 Wuppertal 1 (DE); GESING, Ernst, R., F., D-4006 Erkrath-Hochdahl (DE); MÜLLER, Klaus-Helmut, D-4000 Düsseldorf 13 (DE); MÜLLER, Peter, D-4018 Langenfeld (DE); FINDEISEN, Kurt, D-5090 Leverkusen 1 (DE); SANTEL, Hans-Joachim, D-5090 Leverkusen 1 (DE); LÜRSSEN, Klaus, D-5060 Bergisch Gladbach 2 (DE); SCHMIDT, Robert, R., D-5060 Bergisch Gladbach 2 (DE)
(86) Internationale Anmeldenummer: EP9301227
(87) Internationale Veröffentlichungsnummer: WO93024482

(56) Entgegenhaltungen:
- EP-A- 0 171 286
- EP-A- 0 177 163
- EP-A- 0 238 070
- EP-A- 0 353 641
- EP-A- 0 409 114
- US-A- 4 515 620
- US-A- 4 743 292
- US-A- 4 842 639
- PATENT ABSTRACTS OF JAPAN vol. 10, no. 78 (C-335)27. M{rz 1986
- PATENT ABSTRACTS OF JAPAN

## Beschreibung

Die Erfindung betrifft neue zweifach heterocyclisch substituierte Sulfonylaminocarbonylverbindungen, mehrere Verfahren zu deren Herstellung und deren Verwendung als Herbizide.

Es ist bekannt, dass bestimmte zweifach heterocyclisch substituierte Sulfamoylhamstoffe, wie z.B. 1-(4,6-Dimethoxy-pyrimidin-2-yl)-3-(1-methyl-4-ethoxycarbonyl-pyrazol-5-yl-aminosulfonyl)-harnstoff, herbizide Eigenschaften aufweisen (vgl. JP-A 60 214 785 - zitiert in Chem. Abstracts 104; 109685u; vgl. auch US-P 4 515 620). Verbindungen aus den genannten Publikationen haben jedoch keine größere Bedeutung erlangt.

Es wurden nun neue zweifach heterocyclisch substituierte Sulfonylaminocarbonylverbindungen der allgemeinen Formel (I), 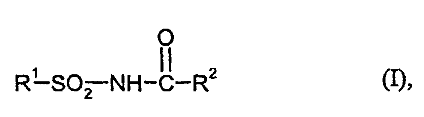 in welcher
- R¹: für einen der nachfolgend genannten, gegebenenfalls substituierten Reste steht: 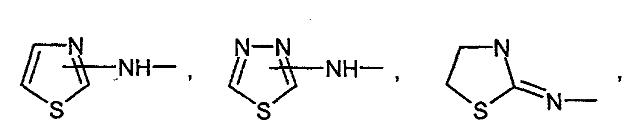 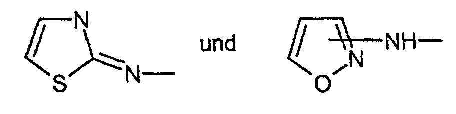 und
- R²: für einen gegebenenfalls substituierten Triazolinon-2-yl-Rest steht, 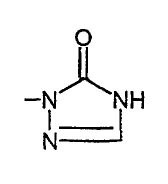
wobei die möglichen Substituenten in R¹ und R² ausgewählt sind aus der Reihe:

Fluor, Chlor, Brom, Iod, Cyano, Nitro, Carboxy, Carbamoyl, Amino, Hydroxy, Formyl, jeweils gegebenenfalls durch Fluor und/oder Chlor substituiertes C₁-C₅-Alkyl, C₅-C₆-Alkandiyl, C₂-C₅-Alkenyl, C₂-C₅-Alkinyl, C₃-C₆-Cycloalkyl, C₃-C₆-Cycloalkyl-C₁-C₃-alkyl, C₁-C₅-Alkoxy, C₂-C₅-Alkenyloxy, C₂-C₅-Alkinyloxy, C₁-C₅-Alkylthio, C₂-C₅-Alkenylthio, C₂-C₅-Alkinylthio, C₁-C₅-Alkylsulfinyl, C₁-C₅-Alkylsulfonyl, C₁-C₅-Alkylamino, Di-(C₁-C₃-alkyl)-amino, C₁-C₅-Alkyl-carbonyl, C₃-C₆-Cycloalkyl-carbonyl, C₁-C₅-Alkoxy-carbonyl, C₁-C₂-Alkoxy-C₁-C₂-alkoxycarbonyl, jeweils gegebenenfalls durch Fluor, Chlor, Brom, Iod, Cyano, Nitro, Carboxy, Carbamoyl, Amino, Hydroxy, Formyl, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl, C₁-C₄-Alkyl-sulfonyl (welche jeweils gegebenenfalls durch Fluor und/oder Chlor substituiert sind) und/oder C₁-C₄-Alkoxy-carbonyl substituiertes Phenoxy
sowie Salze der Verbindungen der Formel (I) gefunden.

Man erhält die neuen zweifach heterocyclisch substituierten Sulfonylaminocarbonylverbindungen der allgemeinen Formel (I) und gegebenenfalls ihre Salze, wenn man
(a) Heterocyclen der allgemeinen Formel (II)

   H-R² (II)

   in welcher
   - R²: die oben angegebene Bedeutung hat,
   mit Chlorsulfonylisocyanat der Formel (III)

   Cl-SO₂-N=C=O (III)

   gegebenenfalls in Gegenwart eines Säureakzeptors und gegebenenfalls in Gegenwart eines Verdünnungsmittels umsetzt (1. Stufe)
   und die hierbei gebildeten Chlorsulfonylaminocarbonylverbindungen der allgemeinen Formel (IV) 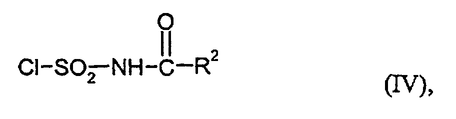 in welcher
   - R²: die oben angegebene Bedeutung hat,
   mit Heterocyclen der allgemeinen Formel (V)

   R¹-H (V),

   in welcher
   - R¹: die oben angegebene Bedeutung hat,
   gegebenenfalls in Gegenwart eines Säureakzeptors und gegebenenfalls in Gegenwart eines Verdünnungsmittels umsetzt (2. Stufe), oder wenn man
(b) Heterocyclen der allgemeinen Formel (V)

   R¹-H (V),

   in welcher
   - R¹: die oben angegebene Bedeutung hat,
   mit Oxysulfonylaminocarbonylverbindungen der allgemeinen Formel (VI) 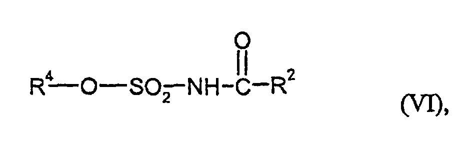 in welcher
   - R²: die oben angegebene Bedeutung hat und
   - R⁴: für Alkyl (vorzugsweise Methyl), Haloalkyl (vorzugsweise Trichlorethyl) oder Aryl (vorzugsweise Phenyl) steht,
   gegebenenfalls in Gegenwart eines Säureakzeptors und gegebenenfalls in Gegenwart eines Verdünnungsmittels umsetzt,
und gegebenenfalls die nach Verfahren (a) oder (b) erhaltenen Verbindungen der Formel (I) nach üblichen Methoden in Salze überführt.

Die neuen zweifach heterocyclisch substituierten Sulfonylaminocarbonylverbindungen der Formel (I) zeichnen sich durch starke herbizide Wirksamkeit aus.

Weitere mögliche Herstellungsmethoden für die erfindungsgemäßen Verbindungen der Formel (I) sind nachstehend aufgeführt, wobei Q, R¹ und R² die oben angegebenen Bedeutungen haben:
(d) Umsetzung von Heterocylen der Formel (V) mit Sulfamoylchlorid (VIII) und dann mit Oxycarbonylverbindungen der Formel (IX), (R⁴: Alkyl, Aryl): 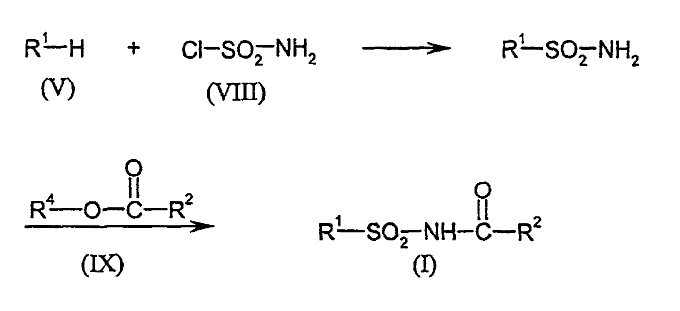
(e) Umsetzung von Heterocyclen der Formel (V) mit Sulfamoylchlorid (VIII) und dann mit Isocyanaten der Formel (X), (Het: Heterocyclyl): 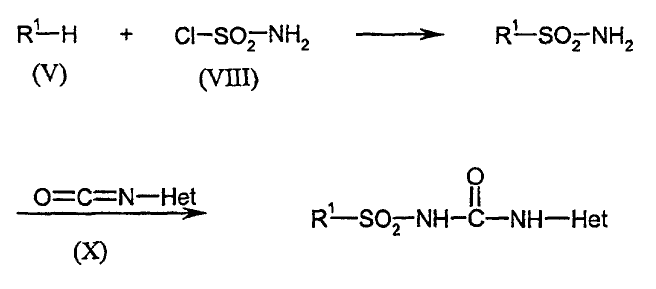
(f) Umsetzung von Heterocyclen mit austauschbaren Gruppen der Formel (XI) mit Sulfamid der Formel (XII) und Urethanen der Formel (XIII), (Het: Heterocyclyl, X¹: Halogen, gegebenenfalls auch Alkylsulfonyl, R⁴: Alkyl, Aryl): 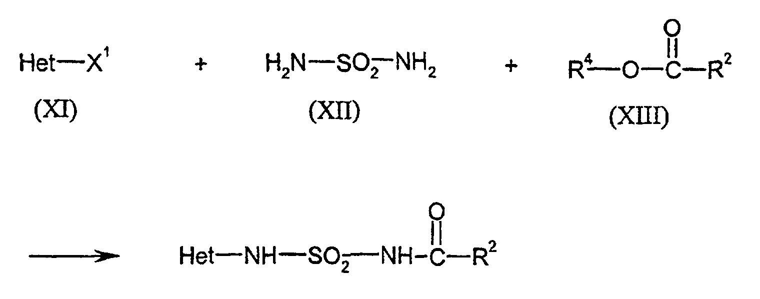
(g) Umsetzung mit Heterocyclen mit austauschbaren Gruppen der Formel (XI) mit Sulfamid der Formel (XII) und Isocyanaten (XIV), (Het: Heterocyclyl, X¹: Halogen, gegebenenfalls auch Methylsulfonyl): 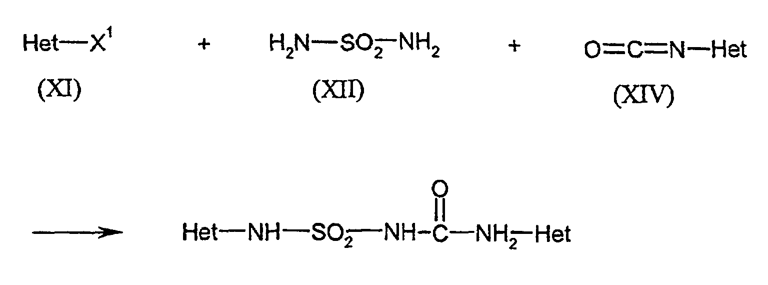
(h) Umsetzung von Heterocyclen mit austauschbaren Gruppen der Formel (XI) mit Sulfamid der Formel (XII), dann mit Chlorameisensäureestern der Formel (XV) und schließlich mit Heterocyclen der Formel (II), (Het: Heterocyclyl, X¹: Halogen, gegebenenfalls auch Alkylsulfonyl, R⁴: Alkyl, Aryl): 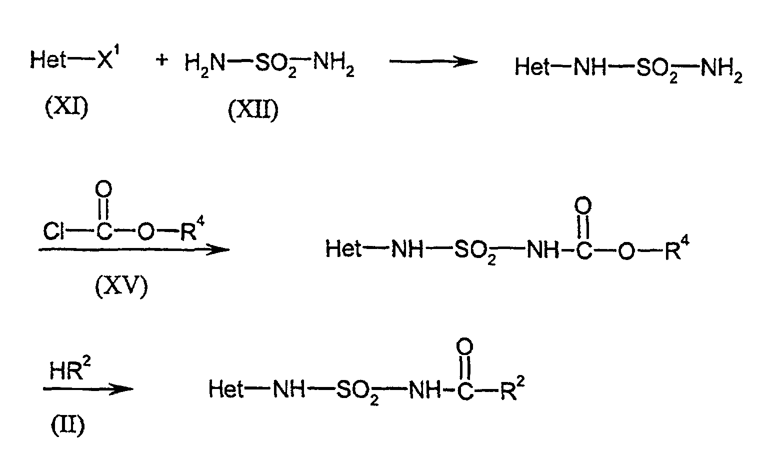
(i) Umsetzung von Heterocyclen der Formel (V) und Carbamoylverbindungen der Formel (XVI) mit Sulfurylchlorid: 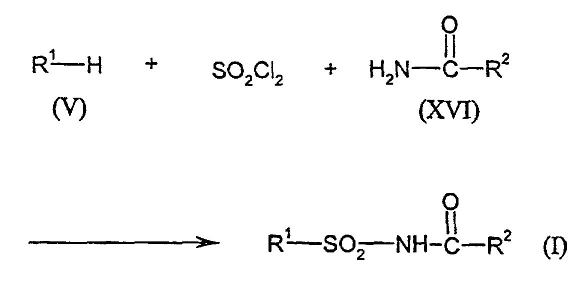
(j) Umsetzung von Sulfonylisocyanaten der Formel (XVII) mit Ammoniak und dann mit Heterocyclylhalogeniden der Formel (XI), (Het: Heterocyclyl, X¹: Halogen, gegebenenfalls auch Alkylsulfonyl): 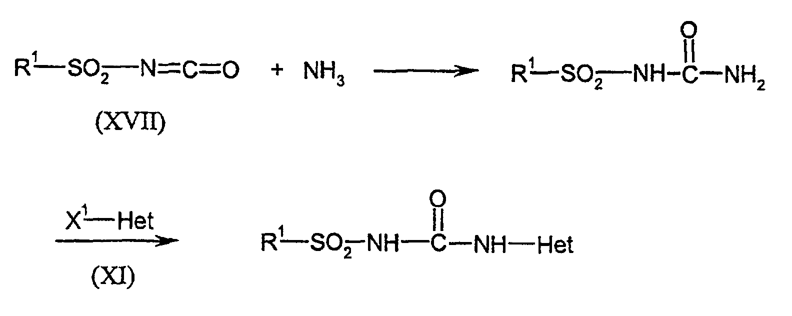
(k) Umsetzung von Chlorsulfonylaminocarbonylverbindungen der Formel (IV) - vgl. Verfahren (a) - mit Ammoniak und dann mit Heterocyclylhalogeniden der Formel (XI), (Het: Heterocyclyl, X¹: Halogen, gegebenenfalls auch Alkylsulfonyl):

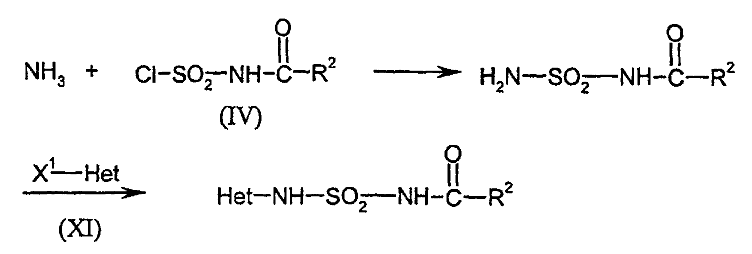

Die Erfindung betrifft weiter vorzugsweise Salze, die man aus Verbindungen der Formel (I) und Basen, wie z.B. Natrium-, Kalium- oder Calcium-hydroxid, -hydrid, -amid oder -carbonat, Natrium- oder Kalium-C₁-C₄-alkanolaten, Ammoniak, C₁-C₄-Alkylaminen, Di-(C₁-C₄-alkyl)-aminen oder Tri-(C₁-C₄-alkyl)-aminen, erhält.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- bzw. Zwischenprodukte. Diese Restedefinitionen können untereinander, also auch zwischen den angegebenen bevorzugten Bereichen beliebig kombiniert werden.

Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Verwendet man beispielsweise 3-Amino-5-methyl-isoxazol und 5-Ethyl-4-methyl-2-phenoxysulfonylaminocarbonyl-2,4-dihydro-3H-1,2,4-triazol-3-on als Ausgangsstoffe, so kann der Reaktionsablauf bei der erfindungsgemäßen Verfahrensvariante (b) durch das folgende Formelschema wiedergegeben werden:

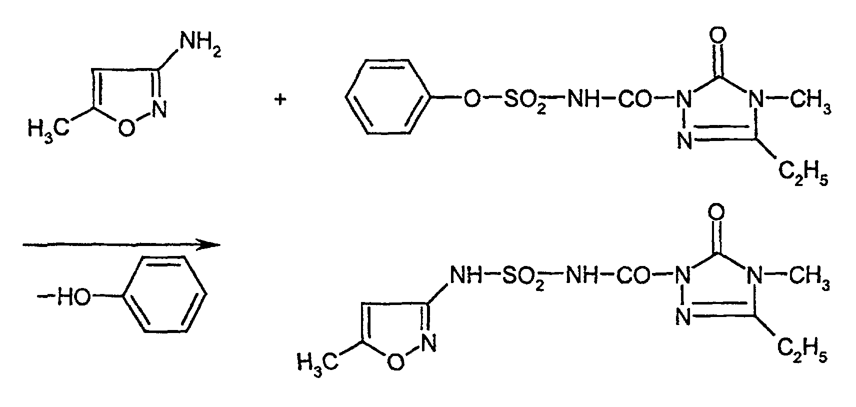

Die für das erfindungsgemäße Verfahren (a) als Ausgangsstoffe benötigten Verbindungen der Formeln (II), (III) und (V) sind bekannte Chemikalien bzw. können nach an sich bekannten Verfahren hergestellt werden (vgl. EP-A 271 833; EP-A 158 594; EP-A 424 849; EP-A 476 554; EP-A 341 489; EP-A 422 469; EP-A 425 948; EP-A 431 291; EP-A 477 646).

Die für das erfindungsgemäße Verfahren (b) als Ausgangsstoffe benötigten Oxysulfonylaminocarbonylverbindungen der Formel (VI) sind ebenfalls bekannt und/oder können nach an sich bekannten Verfahren hergestellt werden (vgl. DE-OS 31 11 451).

Die erfindungsgemäßen Verfahren (a) und (b) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei den erfindungsgemäßen Verfahren (a) und (b) alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und Erdalkalimetall-carbonate und -hydrogencarbonate, wie Natriumund Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert.-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a) und (b) in einem größeren Bereich variiert werden. Im allgemeinen arbeitet man bei Temperaturen zwischen -70°C und +100°C, vorzugsweise bei Temperaturen zwischen -20°C und +50°C.

Die erfindungsgemäßen Verfahren (a) und (b) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a) und (b) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren jeweils nach üblichen Methoden.

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Verdünnungsmittel, wie z.B. Methylenchlorid, Aceton, tert.-Butyl-methylether oder Toluol, und Zugabe einer geeigneten Base. Die Salze können dann - gegebenenfalls nach längerem Rühren - durch Einengen oder Absaugen isoliert werden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapsis, Lepidium, Galium, Stellaria, Matricaria, Anthemis, Glainsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Schccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen, wie z.B. in Getreide, vor allem im Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspension-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

### Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglycolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in die Formulierungen Haftmittel wie Carboxymethylcellulose, natürliche und synthetische pulvrige, körnige oder latexförmige Polymere verwendet werden, wie Gummiarabicum, Polyvinylakohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazollinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulfphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiel

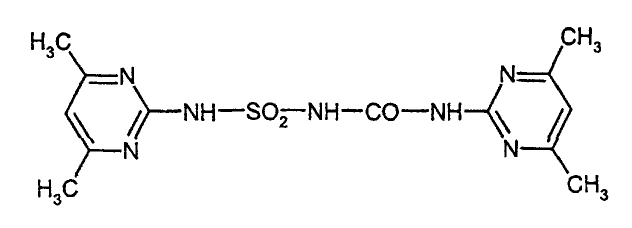

### (Verfahren (a))

7,1 g (0,05 mol) Chlorsulfonylisocyanat werden bei 22°C unter Rühren zu einer Lösung von 6,2 g (0,05 mol) 2-Amino-4,6-dimethyl-pyrimidin und 5,0 g (0,055 mol) Triethylamin in 200 ml Methylenchlorid tropfenweise gegeben und das Gemisch wird noch 30 Minuten bei 22°C gerührt. Dann werden weitere 6,2 g (0,05 mol) 2-Amino-4,6-dimethyl-pyrimidin dazugegeben und die Mischung wird 18 Stunden bei 22°C gerührt. Dann wird abgesaugt und das ungelöste Produkt durch Absaugen isoliert

Man erhält 8,4 g (48 % der Theorie) 1-(4,6-Dimethyl-pyrimidin-2-yl)-3-(4,6-dimethyl-pyrimidin-2-yl-amino-sulfonyl)-harnstoff vom Schmelzpunkt 198°C.

Analog zu dem nicht erfindungsgemäßen Herstellungsbeispiel sowie entsprechend der allgemeinen Beschreibung der erfindungsgemäßen Verfahren können beispielsweise auch die in der nachstehenden Tabelle 1 aufgeführten Verbindungen der Formel (I) hergestellt werden.

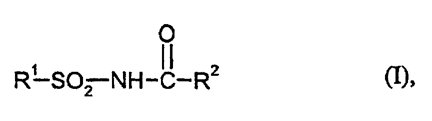

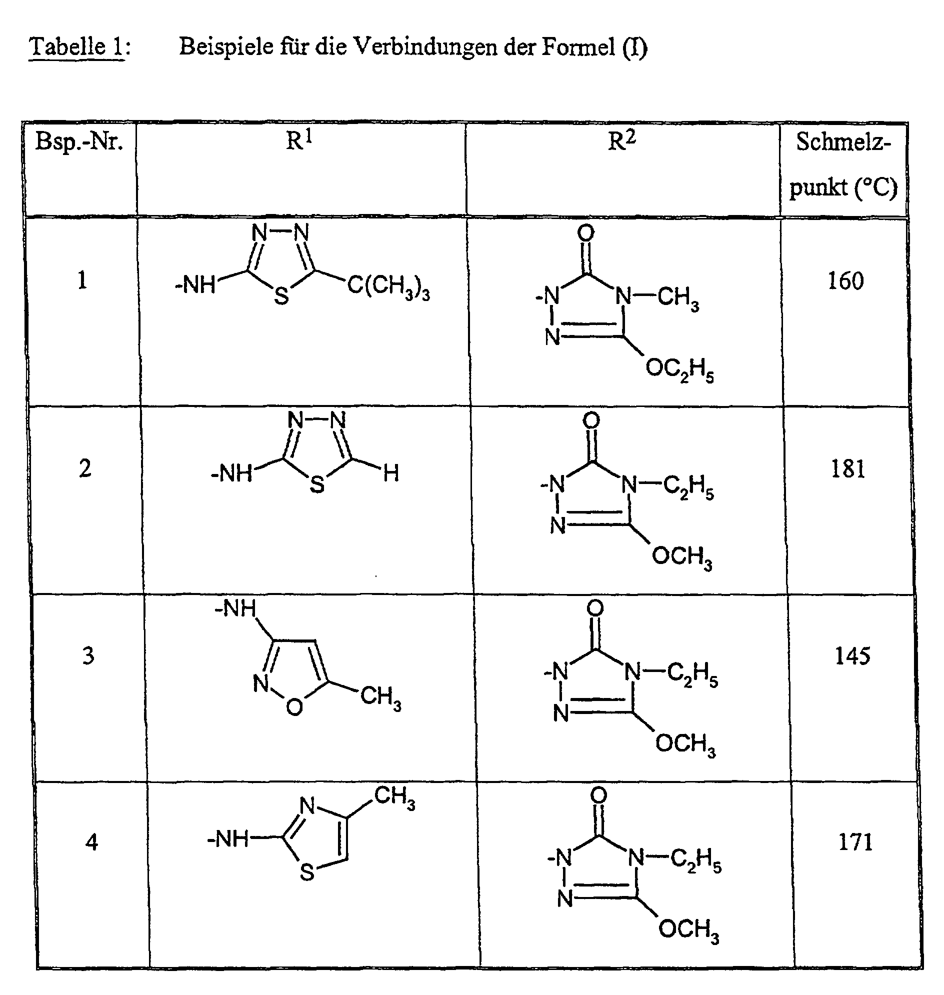

### Beispiel B

Pre-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aurwandmenge des Wirkstoffs pro Flächeneinheit. Nach 3 Wochen wird der Schädigungsgrad der Pflanzen bonitiert in % Schädigung im Vergleich zur Entwicklung der unbehandelten Kontrolle. Es bedeuten:
- 0 %: = keine Wirkung (wie unbehandelte Kontrolle)
- 100 %: = totale Vernichtung

In diesem Test zeigen die erfindungsgemäßen Verbindungen sehr starke Wirkung gegen Unkräuter.

## Patentansprüche

1. Zweifach heterocyclisch substituierte Sulfonylaminocarbonylverbindungen der allgemeinen Formel (I), 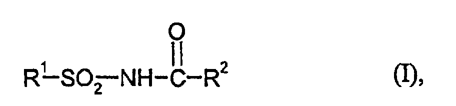 in welcher
R¹ für einen der nachfolgend genannten, gegebenenfalls substituierten Reste steht: 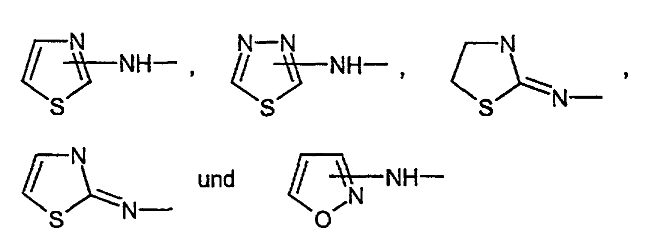 und
R² für einen gegebenenfalls substituierten Triazolinon-2-yl-Rest steht, 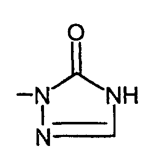 wobei die möglichen Substituenten in R¹ und R² ausgewählt sind aus der Reihe:
Fluor, Chlor, Brom, Iod, Cyano, Nitro, Carboxy, Carbamoyl, Amino, Hydroxy, Formyl, jeweils gegebenenfalls durch Fluor und/oder Chlor substituiertes C₁-C₅-Alkyl, C₅-C₆-Alkandiyl, C₂-C₅-Alkenyl, C₂-C₅-Alkinyl, C₃-C₆-Cycloalkyl, C₃-C₆-Cycloalkyl-C₁-C₃-alkyl, C₁-C₅-Alkoxy, C₂-C₅-Alkenyloxy, C₂-C₅-Alkinyloxy, C₁-C₅-Alkylthio, C₂-C₅-Alkenylthio, C₂-C₅-Alkinylthio, C₁-C₅-Alkylsulfinyl, C₁-C₅-Alkylsulfonyl, C₁-C₅-Alkylamino, Di-(C₁-C₃-alkyl)-amino, C₁-C₅-Alkyl-carbonyl, C₃-C₆-Cycloalkyl-carbonyl, C₁-C₅-Alkoxy-carbonyl, C₁-C₂-Alkoxy-C₁-C₂-alkoxycarbonyl, jeweils gegebenenfalls durch Fluor, Chlor, Brom, Iod, Cyano, Nitro, Carboxy, Carbamoyl, Amino, Hydroxy, Formyl, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl, C₁-C₄-Alkyl-sulfonyl (welche jeweils gegebenenfalls durch Fluor und/oder Chlor substituiert sind) und/oder C₁-C₄-Alkoxy-carbonyl substituiertes Phenoxy
sowie Salze der Verbindungen der Formel (I).

2. Verfahren zur Herstellung von zweifach heterocyclisch substituierten Sulfonylaminocarbonylverbindungen der Formel (I) gemäß Anspruch 1, **dadurch gekennzeichnet, dass** man
(a) Heterocyclen der allgemeinen Formel (II)
H-R² (II)
in welcher
R² die in Anspruch 1 angegebene Bedeutung hat,
mit Chlorsulfonylisocyanat der Formel (III)
Cl-SO₂-N=C=O (III)
gegebenenfalls in Gegenwart eines Säureakzeptors und gegebenenfalls in Gegenwart eines Verdünnungsmittels umsetzt (1. Stufe)
und die hierbei gebildeten Chlorsulfonylaminocarbonylverbindungen der allgemeinen Formel (IV) 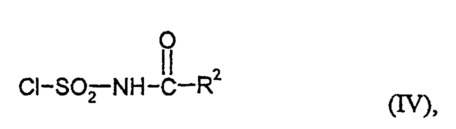 in welcher
R² die in Anspruch 1 angegebene Bedeutung hat,
mit Heterocyclen der allgemeinen Formel (V)
R¹-H (V),
in welcher
R¹ die in Anspruch 1 angegebene Bedeutung hat,
gegebenenfalls in Gegenwart eines Säureakzeptors und gegebenenfalls in Gegenwart eines Verdünnungsmittels umsetzt (2. Stufe), oder wenn man
(b) Heterocyclen der allgemeinen Formel (V)
R¹-H (V),
in welcher
R¹ die in Anspruch 1 angegebene Bedeutung hat,
mit Oxysulfonylaminocarbonylverbindungen der allgemeinen Formel (VI) 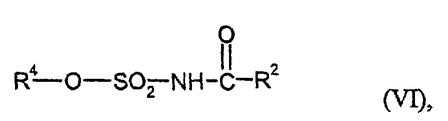
in welcher
R² die in Anspruch 1 angegebene Bedeutung hat und
R⁴ für Alkyl (vorzugsweise Methyl), Haloalkyl (vorzugsweise Trichlorethyl) oder Aryl (vorzugsweise Phenyl) steht,
gegebenenfalls in Gegenwart eines Säureakzeptors und gegebenenfalls in Gegenwart eines Verdünnungsmittels umsetzt,
und gegebenenfalls die nach Verfahren (a) oder (b) erhaltenen Verbindungen der Formel (I) nach üblichen Methoden in Salze überführt.

3. Herbizide Mittel, **gekennzeichnet durch** einen Gehalt an mindestens einer Verbindung der Formel (I) gemäß Anspruch 1.

4. Verwendung von Verbindungen der allgemeinen Formel (I) gemäß Anspruch 1 zur Bekämpfung von unerwünschtem Pflanzenwachstum.

5. Verfahren zur Bekämpfung von Unkräutern, **dadurch gekennzeichnet, dass** man Verbindungen der allgemeinen Formel (I) gemäß Anspruch 1 auf die Unkräuter oder ihren Lebensraum einwirken läßt.

6. Verfahren zur Herstellung von herbiziden Mitteln, **dadurch gekennzeichnet, dass** man Verbindungen der allgemeinen Formel (I) gemäß Anspruch 1 mit Streckmitteln und/oder oberflächenaktiven Mitteln vermischt.

7. Chlorsulfonylaminocarbonylverbindungen der Formel (V) 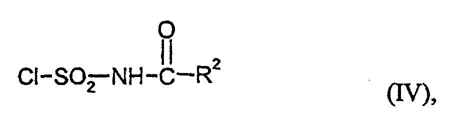 in welcher
R² sowie die möglichen Substituenten in R² die in Anspruch 1 angegebenen Bedeutungen haben.

8. Verbindungen gemäß Anspruch 1, bei denen die im folgenden tabellarisch aufgelisteten Kombinationen der Reste R¹ und R² vorliegen:

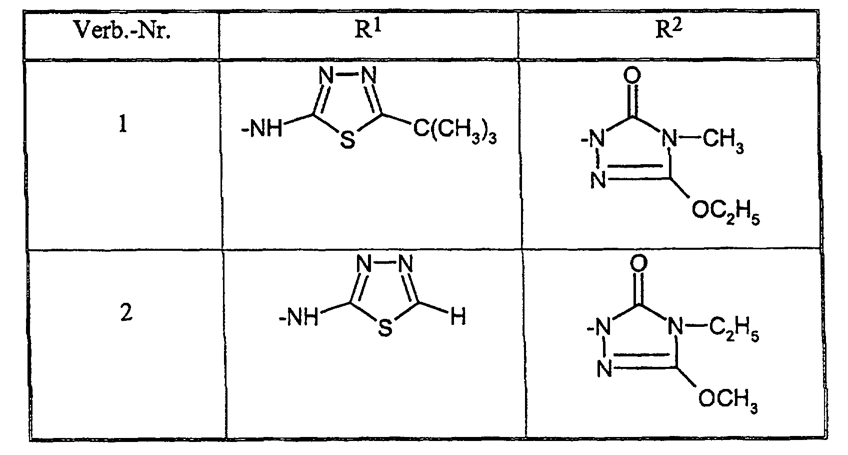

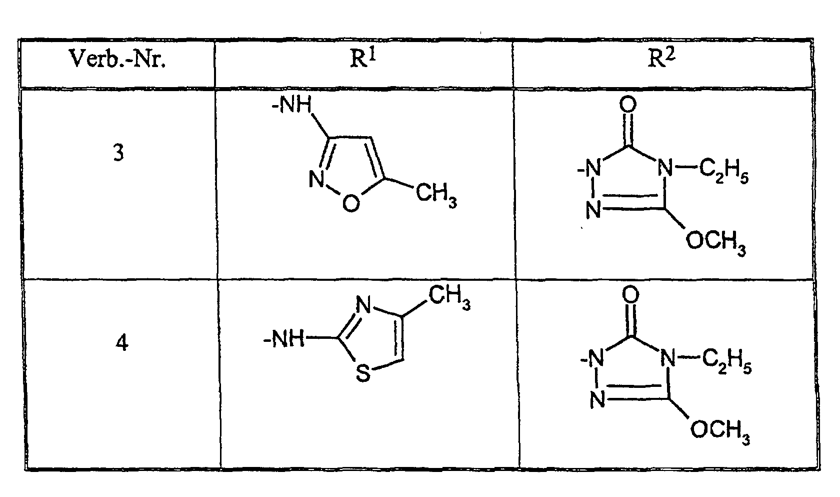

## Claims

1. Heterocyclically disubstituted sulphonylaminocarbonyl compounds of the general formula (I) 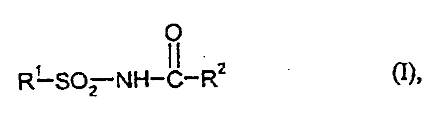 in which
R¹ represents one of the optionally substituted radicals mentioned below 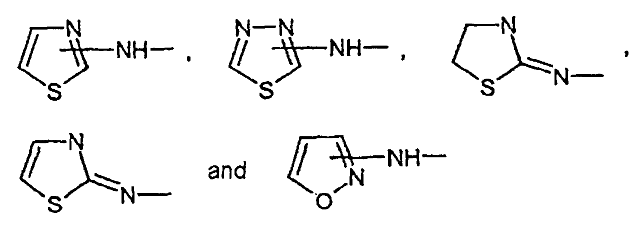 and
R² represents an optionally substituted triazolinon-2-yl radical 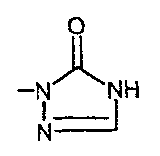 where the substituents which are possible for R¹ and R² are selected from the series comprising:
fluorine, chlorine, bromine, iodine, cyano, nitro, carboxyl, carbamoyl, amino, hydroxyl, formyl, or C₁-C₅-alkyl, C₅-C₆-alkanediyl, C₂-C₅-alkenyl, C₂-C₅-alkinyl, C₃-C₆-cycloalkyl, C₃-C₆-cycloalkyl-C₁-C₃-alkyl, C₁-C₅-alkoxy, C₂-C₅-alkenyloxy, C₂-C₅-alkinyloxy, C₁-C₅-alkylthio, C₂-C₅-alkenylthio, C₂-C₅-alkinylthio, C₁-C₅-alkylsulphinyl, C₁-C₅-alkylsulphonyl, C₁-C₅-alkylamino, di-(C₁-C₃-alkyl)-amino, C₁-C₅-alkyl-carbonyl, C₃-C₆-cycloalkyl-carbonyl, C₁-C₅-alkoxy-carbonyl, C₁-C₂-alkoxy-C₁-C₂-alkoxycarbonyl, each of which is optionally substituted by fluorine and/or chlorine, or phenoxy, which is optionally substituted by fluorine, chlorine, bromine, iodine, cyano, nitro, carboxyl, carbamoyl, amino, hydroxyl, formyl, C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl, C₁-C₄-alkylsulphonyl (each of which is optionally substituted by fluorine and/or chlorine) and/or C₁-C₄-alkoxycarbonyl,
and salts of the compounds of the formula (I).

2. Process for the preparation of heterocyclically disubstituted sulphonylaminocarbonyl compounds of the formula (I) according to Claim 1, **characterized in that**
(a)heterocycles of the general formula (II)
H-R² (II)
in which
R² has the meaning given in Claim 1
are reacted with chlorosulphonyl isocyanate of the formula (III)
Cl-SO₂-N=C=O (III)
if appropriate in the presence of an acid acceptor and if appropriate in the presence of a diluent (step 1),
and the resulting chlorosulphonylaminocarbonyl compounds of the general formula (IV) 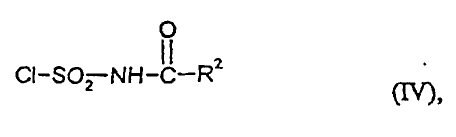 in which
R² has the meaning given in Claim 1,
are reacted with heterocycles of the general formula (V)
R¹-H (V)
in which
R¹ has the meaning given in Claim 1,
if appropriate in the presence of an acid acceptor and if appropriate in the presence of a diluent (step 2), or **in that**
(b) heterocycles of the general formula (V)
R¹-H (V)
in which
R¹ has the meaning given in Claim 1,
are reacted with oxysulphonylaminocarbonyl compounds of the general formula (VI) 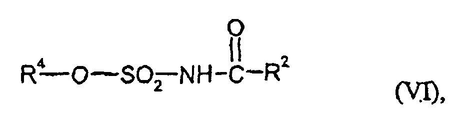 in which
R² has the meaning given in Claim 1 and
R⁴ represents alkyl (preferably methyl), haloalkyl (preferably trichloroethyl) or aryl (preferably phenyl),
if appropriate in the presence of an acid acceptor and if appropriate in the presence of a diluent,
and, if appropriate, the compounds of the formula (I) obtained by process (a) or (b) are converted into salts by customary methods.

3. Herbicidal compositions, **characterized in that** they contain at least one compound of the formula (I) according to Claim 1.

4. Use of compounds of the general formula (I) according to Claim 1 for combating undesired vegetation.

5. Method of combating weeds, **characterized in that** compounds of the general formula (I) according to Claim 1 are allowed to act on the weeds or their environment.

6. Process for the preparation of herbicidal compositions, **characterized in that** compounds of the general formula (I) according to Claim 1 are mixed with extenders and/or surfactants.

7. Chlorosulphonylaminocarbonyl compounds of the formula (V) 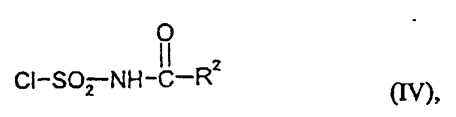 in which
R² and the substituents which are possible for R² have the meanings given in Claim 1.

8. Compounds according to Claim 1, in which the combinations of radicals R¹ and R² listed below in table form are present:

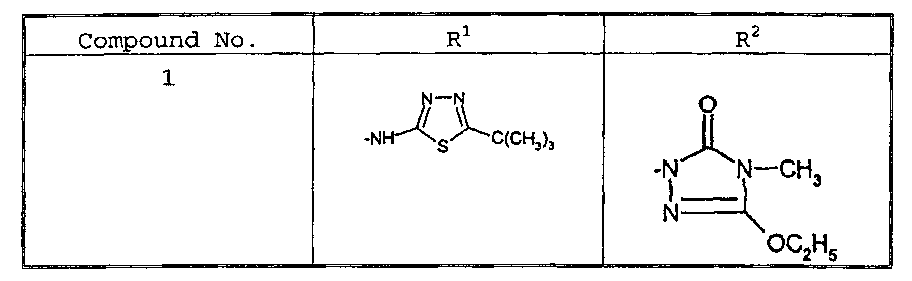

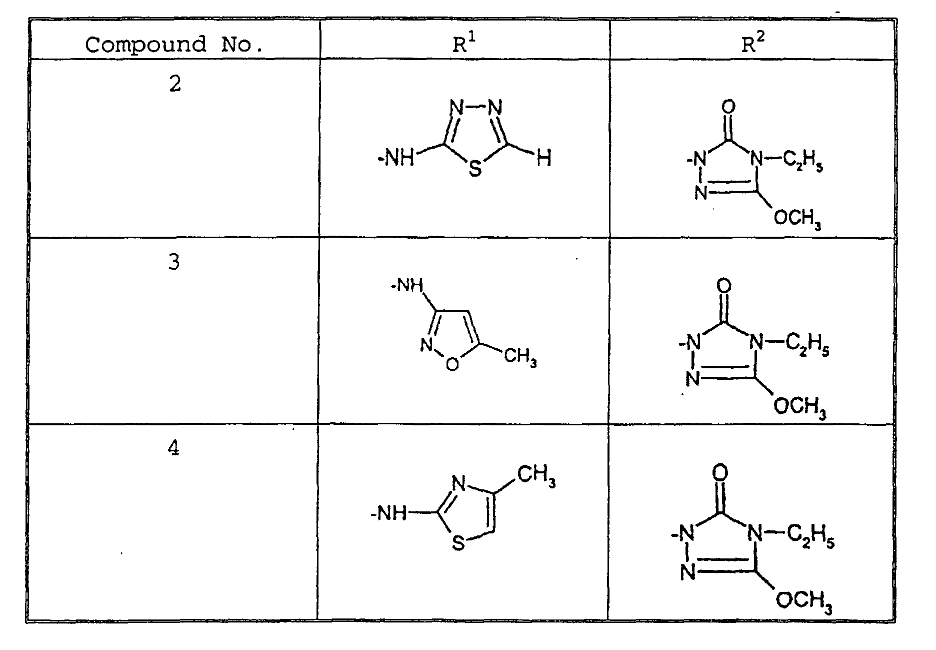

## Revendications

1. Composés sulfonylaminocarbonyle bisubstitués par des hétérocycles, de formule générale (I) : 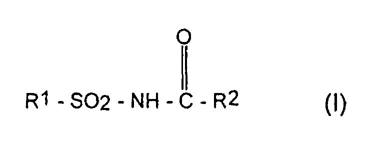 dans laquelle :
R¹ représente l'un des restes indiqués ci-dessous, éventuellement substitués : 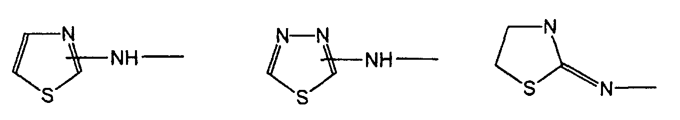 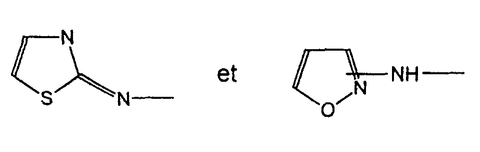
R² représente un reste triazolinon-2-yle éventuellement substitué : 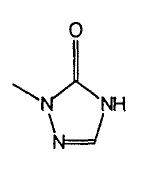
où les substituants possibles de R¹ et R² sont choisis parmi la série :
les atomes de fluor, de chlore, de brome, d'iode, les radicaux cyano, nitro, carboxy, carbamoyle, amino, hydroxy, formyle, les radicaux alcoyle en C₁-C₅, alcanediyle en C₅-C₆, alcényle en C₂-C₅, alcynyle en C₂-C₅, cycloalcoyle en C₃-C₆, (cycloalcoyle en C₃-C₆) alcoyle en C₁-C₃, alcoxy en C₁-C₅, alcényloxy en C₂-C₅, alcynyloxy en C₂-C₅, alcoylthio en C₁-C₅, alcénylthio en C₂-C₅, alcynylthio en C₂-C₅, alcoylsulfinyle en C₁-C₅, alcoylsulfonyle en C₁-C₅, (alcoyl en C₁-C₅) amino, di(alcoyl en C₁-C₃)amino, (alcoyl en C₁-C₅)carbonyle, (cycloalcoyl en C₃-C₆)carbonyle, (alcoxy en C₁-C₅)carbonyle, (alcoxy en C₁-C₂)(alcoxy en C₁-C₂)carbonyle chaque fois éventuellement substitués par les atomes de fluor et/ou de chlore, le radical phénoxy éventuellement substitué par les atomes de fluor, de chlore, de brome, d'iode, les radicaux cyano, nitro, carboxy, carbamoyle, amino, hydroxy, formyle, alcoyle en C₁-C₄ (qui est éventuellement substitué par les atomes de fluor et/ou de chlore), alcoxy en C₁-C₄ (qui est éventuellement substitué par les atomes de fluor et/ou de chlore), alcoylthio en C₁-C₄, alcoylsulfinyle en C₁-C₄, alcoylsulfonyle en C₁-C₄ (qui sont éventuellement substitués par les atomes de fluor et/ou de chlore) et/ou (alcoxy en C₁-C₄) carbonyle,
ainsi que les sels des composés de formule (I).

2. Procédé de préparation de composés sulfonylaminocarbonyle bisubstitués par des hétérocycles, de formule (I) suivant la revendication 1, **caractérisé en ce que**
(a) on fait réagir les hétérocycles de formule générale (II) :
H-R² (II)
dans laquelle :
R² a la signification indiquée à la revendication 1,
avec l'isocyanate de chlorosulfonyle de formule (III) :
Cl-SO₂-N=C=O (III)
le cas échéant, en présence d'un accepteur d'acide et le cas échéant, en présence d'un agent de dilution (étape 1),
et on fait réagir les composés chlorosulfonylaminocarbonyle ainsi formés, de formule générale (IV) : 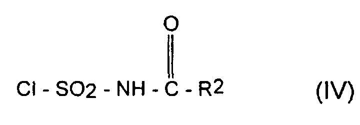 dans laquelle:
R² a la signification indiquée à la revendication 1,
avec les hétérocycles de formule générale (V) :
R¹-H (V)
dans laquelle :
R¹ a la signification indiquée à la revendication 1,
le cas échéant, en présence d'un accepteur d'acide et le cas échéant, en présence d'un agent de dilution (étape 2), ou lorsque
(b) on fait réagir les hétérocycles de formule générale (V) :
R¹-H (V)
dans laquelle :
R¹ a la signification indiquée à la revendication 1,
avec les composés oxysulfonylaminocarbonyle de formule générale (VI) : 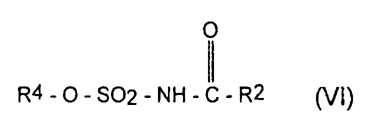 dans laquelle :
R² a la signification indiquée à la revendication 1, et
R⁴ représente les radicaux alcoyle (de préférence, méthyle), haloalcoyle (de préférence, trichloroéthyle) ou aryle (de préférence, phényle),
le cas échéant, en présence d'un accepteur d'acide et le cas échéant, en présence d'un agent de dilution,
et le cas échéant, les composés de formule (I), obtenus d'après le procédé (a) ou (b), sont convertis en sels par les procédés usuels.

3. Agent herbicide, **caractérisé par** la présence d'au moins un composé de formule (I) suivant la revendication 1.

4. Utilisation des composés de formule générale (I) suivant la revendication 1, pour lutter contre la croissance de plantes non désirées.

5. Procédé pour lutter contre les mauvaises herbes, **caractérisé en ce que** l'on fait agir des composés de formule générale (I) suivant la revendication 1, sur les mauvaises herbes ou leur site de développement.

6. Procédé de préparation d'agents herbicides, **caractérisé en ce que** l'on mélange des composés de formule générale (I) suivant la revendication 1, avec des diluants et/ou des agents tensioactifs.

7. Composés chlorosulfonylaminocarbonyle de formule (IV) : 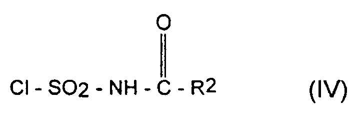 dans laquelle:
R² ainsi que les substituants possibles sur R² ont les significations indiquées à la revendication 1.

8. Composés suivant la revendication 1, qui présentent les combinaisons indiquées dans le tableau suivant, des restes R¹ et R² :
